(19) Europäisches Patentamt
European Patent Office
Office européen des brevets

(11) Veröffentlichungsnummer: **0 198 227**
**A2**

(12) # EUROPÄISCHE PATENTANMELDUNG

(21) Anmeldenummer: **86103390.0**

(22) Anmeldetag: **13.03.86**

(51) Int. Cl.⁴: **C07D 471/04**, C07D 495/14, A61K 31/435, //(C07D471/04,235:00,221:00),(-C07D495/14,333:00,235:00,221-:00)

(30) Priorität: **20.03.85 DE 3510044**
**28.12.85 DE 3546244**

(43) Veröffentlichungstag der Anmeldung:
**22.10.86 Patentblatt 86/43**

(84) Benannte Vertragsstaaten:
**AT BE CH DE FR GB IT LI LU NL SE**

(71) Anmelder: **BOEHRINGER INGELHEIM KG**

**D-6507 Ingelheim am Rhein(DE)**
Anmelder: **Boehringer Ingelheim International G.m.b.H**

**D-6507 Ingelheim am Rhein(DE)**

(72) Erfinder: **Lösel, Walter, Dr.**
**Im Herzenacker 26**
**D-6535 Gau-Algesheim(DE)**
Erfinder: **Roos, Otto, Dr.**
**Elsheimer Strasse 36**
**D-6501 Schwabenheim(DE)**
Erfinder: **Schnorrenberg, Gerd, Dr.**
**Ernst-Ludwig-Strasse 66a**
**D-6535 Gau-Algesheim(DE)**
Erfinder: **Arndts, Dietrich, Dr.**
**Mühlstrasse 7**
**D-6531 Appenheim(DE)**
Erfinder: **Hinzen, Dieter, Prof. Dr.**
**Konrad-Adenauer-Strasse 26**
**D-6501 Zornheim(DE)**
Erfinder: **Kuhn, Franz-Josef, Dr.**
**Beethovenstrasse 11**
**D-6535 Gau-Algesheim(DE)**
Erfinder: **Lehr, Erich, Dr.**
**In der Toffel 5**
**D-6531 Waldalgesheim(DE)**
Erfinder: **Reichl, Richard, Dr.**
**Im Hippel 55**
**D-6535 Gau-Algesheim(DE)**
Erfinder: **Streller, Ilse, Dr.**
**Waldstrasse 16**
**D-6534 Stromberg(DE)**
Erfinder: **Speck, Georg, Dr.**
**Rheinstrasse 13**
**D-6507 Ingelheim/Rhein(DE)**



Rank Xerox

(54) **Imidazo-Isochinoline und Imidazo-Thienopyridin-Verbindungen, Arzneimittel enthaltend diese Verbindungen und Verfahren zu ihrer Herstellung.**

(57) Verbindungen der allgemeinen Formel

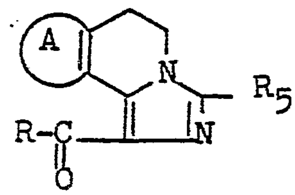

(A und R sind in der Beschreibung näher erläutert) und ihre Säureadditionssalze eignen sich zur Verwendung als Arzneistoffe, insbesondere in antiasthmatischen pharmazeutischen Präparaten.

Rank Xerox

# Imidazo-Isochinoline und Imidazo-Thienopyridin-Verbindungen, Arzneimittel enthaltend diese Verbindungen und Verfahren zu ihrer Herstellung

Die Erfindung betrifft Verbindungen der allgemeinen Formel I

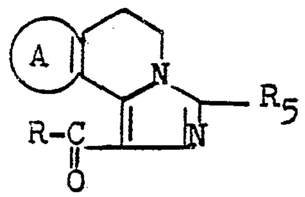

I

und ihre Säureadditionssalze,

In der allgemeinen Formel I

A eine Gruppe bedeutet, die zusammen mit den zwei Kohlenstoffatomen des Pyridylsystems, an welche sie gebunden ist, eine ein-oder mehrfach substituierte Phenyl-oder eine ein-oder mehrfach substituierte Thienogruppe bildet, wobei der oder die Substituenten eine $C_1$-$C_3$Alkoxy, Hydroxy oder Methansulfonyloxy Gruppe darstellen,

$R_5$ bedeutet eine $C_1$-$C_4$Alkylgruppe, vorzugsweise Methyl

R bedeutet
- (i) -OH,
- (ii) eine gesättigte oder ungesättigte, verzweigte oder unverzweigte $C_1$-$C_5$Alkoxygruppe; oder
- (iii) -$NR_1R_2$, worin $R_1$ und $R_2$ unabhängig voneinander stehen für
  - a) Wasserstoff;
  - b) eine gesättigte oder ungesättigte, verzweigte oder unverzweigte Kohlenwasserstoffgruppe mit 1 bis 8 Kohlenstoffatom-(en) (gegebenenfalls ein oder mehrfach substituiert durch Halogen, Hydroxy, Methoxy, Amino, Methylamino, Anilino, Dimethylamino, $C_3$-$C_7$ Cycloalkyl, Phenyl - (gegebenenfalls ein-oder mehrfach substituiert durch Halogen oder Methoxy) oder einen heterocyclischen 5-bis 7-Ring, der ein oder zwei gleiche oder verschiedene Heteroatome, aus der Gruppe N, S und O enthält, und gegebenenfalls durch ($C_1$-$C_3$)Alkyl substituiert ist);
  - c) eine $C_3$-$C_7$ Cycloalkylgruppe;
  - d) Phenyl, gegebenenfalls ein oder mehrfach durch Halogen oder Methoxy substituiert:
  - e) einen heterocyclischen 5-bis 7-Ring, der ein oder zwei gleich oder verschiedene Heteroatome, aus der Gruppe N, S oder O enthält;
  - f) Amino substituiert durch einen heterocyclischen 5-bis 7-Ring, der ein oder zwei gleiche oder verschiedene Heteroatome, aus der Gruppe N, S oder O enthält, und der gegebenenfalls ein oder mehrfach durch Halogen substituiert ist; oder $R_1$ und $R_2$ bilden zusammen mit dem Stickstoffatom einen 5-oder 6-Ring, der gegebenenfalls ein Sauerstoffatom oder ein weiteres Stickstoffatom als

Ringheteroatom enthält und der seinerseits durch einen 5-oder 6-gliedrigen homo-oder heterocyclischen Ring, der ein oder zwei gleiche oder verschiedene Heteroatome, aus der Gruppe N, S oder O, enthält, substituiert sein kann.

Des Ausdruck "Halogen" bedeutet ein Fluor-, Chlor-, Brom-oder Jodatom.

Bevorzugt sind Verbindungen der allgemeinen Formel II

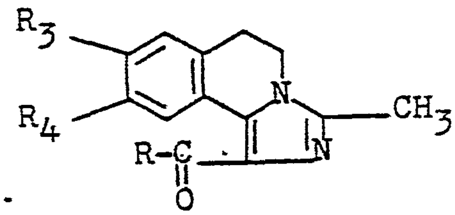

II

worin

$R_3$ und $R_4$ unabhängig voneinander Wasserstoff, Hydroxy, Methoxy und Methansulfonyloxy bedeuten und R die oben angegebe Bedeutung hat, und ihre Säureadditionssalze.

In den Verbindungen der allgemeinen Formel II sind die folgenden Kombinationen von $R_3$ und $R_4$ sind bevorzugt,

$R_3 = R_4 =$ Methoxy

$R_3 =$ Methoxy, $R_4 =$ Hydroxy oder umgekehrt,

$R_3 =$ Methoxy, $R_4 =$ Methansulfonyloxy oder umgekehrt,

$R_3 =$ Methoxy, $R_4 =$ Wasserstoff oder umgekehrt.

Hervorzuheben sind auch Verbindungen der allgemeinen Formel III und IV

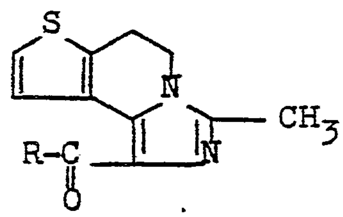

III

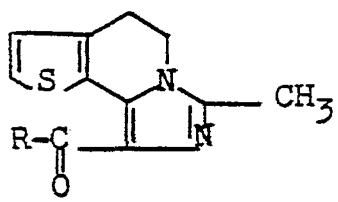

IV

und ihre Säureadditionssalze.

Falls R eine $C_1$-$C_5$-Alkoxygruppe ist, so ist diese vorzugsweise Methoxy oder Ethoxy.

Falls R eine -$NR_1R_2$-Gruppe ist, so sind die Substituenten $R_1$ und $R_2$ vorzugsweise wie folgt;

$R_1 =$ Wasserstoff, und

$R_2 = C_1$-$C_5$-Alkyl, $C_3$-$C_5$-Alkenyl, $C_3$-$C_5$-Alkinyl, $C_3$-$C_6$-Cycloalkyl ($C_1$-$C_5$)-Alkyl oder $C_3$-$C_6$-Cycloalkyl, z.B.:

Methyl, Ethyl, n-Propyl, n-Butyl, n-Pentyl, Isopropyl, Isobutyl, Isopentyl,

Allyl

Propinyl,

Cycloalkyl, Cyclohexyl oder

Cyclohexylmethyl; ist

oder $R_1$ und $R_2$ je für eine ($C_1$-$C_2$)Alkylgruppe stehen;

oder

$R_1 =$ Wasserstoff

$R_2 =$ eine $C_1$-$C_5$Alkylgruppe substituiert durch

a) einen heterocyclischen 5-oder 6-Ring mit 1 bis 5 Kohlenstoffatomen enthaltend zumindest eine Doppelbindung und mindestens ein Heteroatom ausgewählt unter N, S und O, z.B. Thienyl, Imidazolyl, Furyl oder Pyridinyl: oder b) einen gegebenenfalls N-Methyl-substituierten heterocyclischen 5-oder 6-Ring ohne Doppelbindung, welche ein O Atom als weiteres Heteroatom enthalten kann, z.B. Morpholinyl, Pyrrolidinyl oder N-Methyl-pyrrolidin-2-yl,

oder

$R_1$ = Wasserstoff

$R_2$ = Phenyl oder Phenyl substituiert durch Halogen,

z.B. 4-Fluorphenyl oder 3-Chlorphenyl,

oder

$R_1$ = Wasserstoff

$R_2$ = $C_1$-$C_5$Alkyl substituiert durch $C_1$-$C_2$-Alkoxy, Halogen, Hydroxy, $C_1$-$C_2$-Dialkylamino, oder Anilino, z.B. Methoxy ($C_2$ oder $C_3$) Alkyl, 2-Hydroxyethyl, 3-Hydroxy-n-propyl, 2-Chlorethyl, 3-Chloro-n-propyl, 2-Dimethylamino-ethyl, 2-Anilino-ethyl und 3-Dimethylamino-n-propyl.

$R_1$ = Wasserstoff

$R_2$ = einen heterocyclischen 5-oder 6-Ring, der mindestens eine Doppelbindung und eine oder zwei gleich oder verschiedene Heteroatome ausgewählt aus der Gruppe N, S oder O enthält

oder $R_1$ und $R_2$ zusammen mit dem Stickstoffatom eine Piperazingruppe bilden, welche durch einen heterocyclischen 6-Ring der ein oder zwei N-Atome enthält N-substituiert ist, z.B.

4-(2-Pyridinyl)-piperazinyl,

4-(2-Pyrazinyl)-piperazinyl oder

4-(2-Pyrimidinyl)-piperazinyl,

oder

$R_1$ und $R_2$ bilden zusammen mit dem Stickstoffatom einen heterocyclischen 5-oder 6-Ring ohne Doppelbindung, welcher gegebenenfalls als weiteres Heteroatom ein N-oder O-Atom enthält, z.B. Pyrrolidinyl oder Morpholinyl.

In der DE-OS 28 21 226 sind Verbindungen der allgemeinen Formel beschrieben

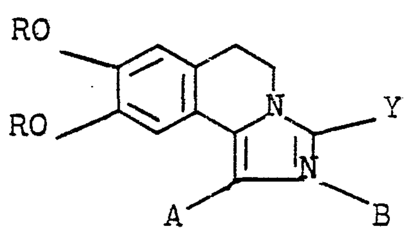

worin z.B. R Wasserstoff oder 1-4 Kohlenstoffatome enthaltendes Alkyl bedeutet,

Y Alkyl darstellt,

B ein einsames Elektronpaar bedeutet und

A Nitril oder Carbamoyl ist.

Nach der Beschreibung der Offenlegungsschrift sind die Verbindungen als starke Bronchus erweiternde Mittel wirksam und können dadurch als Wirkstoff von antiasthmatischen pharmazeutischen Präparaten verwendet werden. Verbindungen in welchen gleichzeitig Y Methyl, A Carbamoyl und B ein einsames Elektronpaar bedeuten, sind nicht genannt.

Ein weiterer Gegenstand der vorliegenden Erfindung besteht daher aus Verbindungen der Formel:

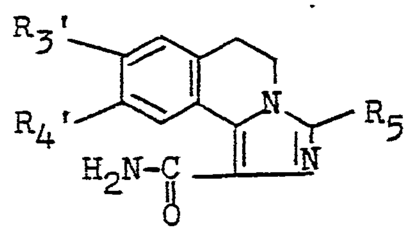

VIII

und ihren Säureadditionssalzen, worin $R_3$' und $R_4$' unabhängig voneinander Hydroxy oder Methoxy bedeuten, und ihrer Verwendung zur Behandlung der hierin erwähnten Indikationen sowie der Herstellung von pharmazeutischen Zubereitungen zur Behandlung solcher Indikationen und ihrer Verwendung zur Behandlung der hierin erwähnten Coronarerkankung und zur Verbesserung der Gewebedurchblutung.

Die erfindungsgemäßen Verbindungen weisen eine deutliche cardioprotective Wirkung auf. Bekanntlich ist der myocardiale $Ca^{2+}$ Gehalt ein Maß für die hypoxische bzw. die durch toxische Catecholamindosen hervorgerufene Herzschädigung - (Higgins et. al. Mol. Cell. Cardiol. 10 , 427-438, 1984; Nakanischi et. al. Am. J. Physiol. 242, 437-439, 1982, Flecktenstein A., Vorträge der Erlanger Physiol. Tagung 1970, Edit. Keidel, Springer Verlag, Berlin, Heidelberg, New York, 1971). Umgekehrt ist die Inhibition der hypoxischen oder Isoprenalin-bedingten myocardialen Calciumaufnahme ein Maß für die cardioprotektive Effektivität von Calciumantagonisten (Fleckenstein s.o.) von Calmoduleninhibitoren (Higgings s.o.) und anderen Pharmaka, z.B. Betaadrenolytika (Arndts, Arzneimittelforschung 25, 1279-1284, 1975).

Die cardioprotektive Wirkung wurde an wachen Ratten nach subcutaner oder peroraler Wirkstoffgabe anhand der von Arndts (s.o.) beschriebenen Methode hergestellt und die Wirkungsstärke der Testsubstanzen als $H_{50}$-Wert angegeben; dieser-Wert entspricht der Dosis, die die durch eine Gabe von 30 mg/kg s.c., Isoprenalin bedingte myocardiale Radiocalciumaufnahme zu 50 % hemmt.

Hier erwiesen sich die untersuchten neuen Verbindungen als bis zu fünfmal wirksamer als das bekannte Handelsprodukt Propranolol.

In-vitro Untersuchungen an der glatten Mukulatur (Aortenstreifen) haben ergeben, daß es sich bei den erfindungsgemäßen Verbindungen um einen Calciumantagonisten mit einem neuen Wirkungsmechanismus handelt.

Aufgrund der obigen Befunde sollen die Verbindungen der allgemeinen Formel I bzw. ihre Säureadditionssalze als Wirkstoff für Arzneimittel gegen Coronarerkrankungen, insbesondere gegen Angina Pectoris oder zur Verbesserung der Ischamie-Toleranz im Myocard wirksam sein.

Daneben verbessern die erfindungsgemäßen Substanzen die Gewebsdurchblutung und die Gewebssauerstoffversorgung besonders im zentralen Nervensystem. In Versuchen mit Einschränkung des Kurzzeitgedächtnisses durch Gabe des muscarin-cholinergen Antagonisten Scopolamin (0,6 mg/kg i.p.) (Psychopharmacology 78, s. 104-111 (1982)) sind die Verbindungen in der Lage, dieser pharmakologisch induzierten cerebralen Insuffizienz entgegenzuwirken bzw. sie aufzuheben.

Bei der Prüfung der Überlebensfähigkeit von Tieren in einer geschlossenen Kammer (Hypoxietoleranztest), welche mit einem Gemisch bestehend aus 96,5 % Stickstoff und 3,5 % Sauerstoff, durchströmt wurde, wiesen die mit den erfindungsgemäßen Substanzen vorbehandelten Tiere eine statistisch hoch signifikant größere Überlebensfähigkeit auf, als Kontrolltiere bzw. mit Diltiazem, Verapamil oder Nifedipine vorbehandelte Tiere.

Die mit dieser Methode geprüfte hirnprotektive Wirkung der Substanzen war bereits bei einer Dosis vom 5 mg/kg p.o. ausgeprägt. Damit sind die erfindungsgemäßen Verbindungen sowohl hinsichtlich der wirksamen Dosis als auch der tierexperimentell erzielten Leistungsverbesserung den genannten bekannten Substanzen deutlich überlegen.

Aufgrund dieser Befunde sollen die Verbindungen der allgemeinen Formel I bzw. ihre Säureadditionssalze als Wirkstoffe für Arzneimittel gegen cerebrale Stoffwechselstörungen bzw. das hirnorganische Psychosyndrom sowie postraumatische und alkoholische Hirnschädigungen Verwendung finden.

Die neuen Verbindungen können allein oder in Kombination mit anderen erfindungsgemäßen Wirkstoffen, gegebenenfalls auch in Kombination mit weiteren pharmakologisch aktiven Wirkstoffen, z.B. weiteren Cerebroaktivatoren zur Anwendung gelangen. Geeignete Anwendungsformen sind beispielsweise Tabletten, Kapseln, Zäpfchen, Lösungen, Säfte, Emulsionen oder dispersible Pulver. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmittel, wie Maisstärke oder Alginsäure, Bindemitteln, wie Stärke oder Gelatine, Schmiermitteln, wie Magnesiumstearat oder Talk, und/oder Mitteln zur Erzielung eines Depoteffektes, wie Carboxypolymethylen, Carboxymethylcellulose, Celluloseacetatphthalat, oder Polyvinylacetat erhalten werden. Die Tabletten können auch aus mehreren Schichten bestehen.

Entsprechend können Dragées durch Überziehen von analog den Tabletten hergestellten Kernen mit üblicherweise in Dragéeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Dragéehülle zur Erzielung eines Depoteffektes aus mehreren Schichten bestehen, wobei die oben bei den Tabletten erwähnten Hilfsstoffe verwendet werden können.

Säfte der erfindungsgemäßen Wirkstoffe bzw. Wirkstoffkombinationen können zusätzlich noch ein Süßungsmittel, wie Saccharin, Cyclamat, Glycerin oder Zucker sowie ein geschmackverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Injektionslösungen werden in üblicher Weise, z.B. unter Zusatz von Konservierungsmitteln, wie p-Hydroxybenzoaten, oder Stabilisatoren, wie Alkalisalzen der Ethylendiamintetraessigsäure hergestellt und in Injektionsflaschen oder Ampullen abgefüllt.

Die eine oder mehrere Wirkstoffe bzw. Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Geeignete Zäpfchen lassen sich beispielsweise durch Vermischen mit dafür vorgesehenen Trägermitteln, wie Neutralfetten oder Polyethylenglykol bzw. dessen Derivaten, herstellen.

Die Verbindungen können sowohl enteral oder auch parenteral verabreicht werden. Als Dosis für die orale Anwendung werden 0,1 bis 10 mg/kg, vorzugsweise 0,2 bis 0,5 mg/kg Wirkstoff pro Dosis, für die parenterale Anwendung 0,01 bis 0,5 mg/kg, vorzugsweise 0,05 bis 0,2 mg/kg pro Dosis vorgeschlagen.

Die erfindungsgemäßen Verbindungen können durch verschiedene Verfahren hergestellt werden, wie unten beschrieben:

VERFAHREN A

(zur Herstellung von Verbindungen der allgemeinen Formel I, in welchen R nicht $-NH_2$ ist)

eine Verbindung der allgemeinen Formel V

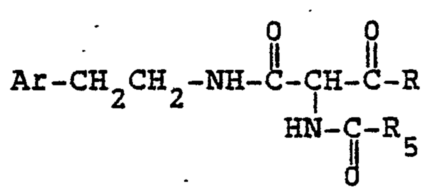

V

worin Ar für eine unsubstituierte oder ein oder mehrfach substituierte Phenylgruppe oder eine unsubstituierte oder ein-oder zweifach substituierte Thienogruppe steht, mit der Maßgabe, daß eine -CH = Gruppe der Phenyl-oder Thienogruppe, die dem Kohlenstoffatom benachbart ist, das die Seitenkette trägt, unsubstituiert ist, und wobei das oder die Substituenten aus der Gruppe $C_1$-$C_3$-Alkoxy, Hydroxy oder Methylsulfonyloxy ausgewählt sind und R die oben angegebenen Bedeutungen hat, wird in Gegenwart eines geeigneten Kondensationsmittels cyclisiert.

Als Kondensationsmittel eignen sich Lewis-Säuren, wie z.B. Phosphoroxychlorid, Phosphorpentachlorid, Zinntetrachlorid, aber auch anorganische Säuren wie Polyphosphorsäure, Siloxan-Polyphosphorsäure-Gemische oder Lösungen von $P_2O_5$ in Methansulfonsäure. Sie werden im allgemeinen im Überschuß eingesetzt. Bevorzugte Kondensationsmittel sind Phosphoroxychlorid, Polyphosphorsäure und Methansulfonsäure /$P_2O_5$ Gemische mit einem $P_2O_5$-Anteil von ca. 10 Gewichtsprozenten.

Die Cyclisierung kann in Gegenwart oder in Abwesenheit eines Lösungsmittels durchgeführt werden. Geeignet sind alle inerten Lösungsmittel, soweit sie eine ausreichende Löslichkeit für die Reaktionspartner besitzen und einen ausreichend hohen Siedepunkt aufweisen, beispielsweise Benzol, Alkylbenzole, Chloroform, Methylenchlorid und Acetonnitril.

Wenn gewünscht kann man das Kondensationsmittel beispielsweise Phosphoroxychlorid, Polyphosphorsäure oder ein Methansulfonsäure-/$P_2O_5$-Gemisch als Lösungsmittel verwenden.

Die Umsetzung kann innerhalb eines großen Temperaturbereiches, vorzugsweise unter Erwärmen oder Erhitzen bis etwa zum Siedepunkt des Lösungsmittels, durchgeführt werden.

VERFAHREN B

(zur Herstellung von Verbindungen der allgemeinen Formel I, worin R -$NR_1R_2$ ist)

Eine Verbindung der allgemeinen Formel VI

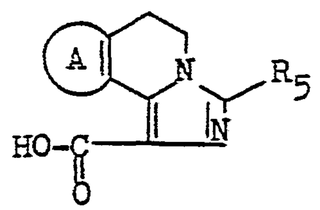

VI

oder ein $C_1$-$C_5$Alkylester oder ein aktiviertes Derivat davon, wird mit einer Verbindung der allgemeinen Formel V

HNR $_1R_2$ VII

umgesetzt.

Geeignete aktivierte Derivate der Verbindung der Formel VI sind beispielsweise Säurehalogenide, Säureazide oder gemischte Säureanhydride (z.B. mit einer aromatischen oder aliphatischen Carbonsäure, Alkylkohlensäure oder Dialkylphosphorsäure usw.), ferner Säureamide (beispielsweise mit Imidazol, 4-substituiertem Imidazol, Dimethylpyrazol, Triazol oder Tetrazol, oder aktivierte Ester, z.B. Cyanomethyl, Methoxymethyl, Vinyl, Propargyl oder p-Nitrophenylester oder Ester mit z.B. Dimethylhydroxylamin, 1-Hydroxysuccinimid, Dicyclohexylharnstoff. Bevorzugt werden die aktiven Imidazolide, sowie die $C_1$-$C_5$Alkyl Ester.

Die Amidbildung wird im allgemeinen in einem inerten Lösungsmittel wie Dioxan, Acetonitril, Dimethylformamid oder einem Gemisch eines oder mehrerer Lösungsmittel, gegebenenfalls in Gegenwart einer organischen oder anorganischen Base als Säurefänger durchgeführt. Es ist aber auch möglich die Reaktion ohne Lösungsmittel mit dem Amin im Überschuß durchzuführen.

Die Reaktionstemperatur kann, je nach eingesetzten Ausgangsstoffen, in weiteren Grenzen variieren und zwischen etwa 0°C und der Siedetemperatur des Reaktionsgemisches liegen.

Die Endprodukte der oben beschriebenen Reaktionen sind Basen und können auf übliche Weise mit anorganischen oder organischen Säuren in beliebige physiologisch unbedenkliche Säureadditionssalze überführt werden.

Zur Salzbildung geeignete Säuren sind beispielsweise Salzsäure, Bromwasserstoffsäure, Schwefelsäure, Phosphorsäure, Salpetersäure, Essigsäure, Propionsäure, Buttersäure, Oxalsäure, Malonsäure, Bernsteinsäure, Maleinsäure, Fumarsäure, Milchsäure, Weinsäure, Zitronensäure, Apfelsäure, Benzoesäure, Zimtsäure, Ascorbinsäure, Methansulfonsäure usw.

Die Ausgangsverbindungen der allgemeinen Formel V sind zum Teil neu. Sie werden hergestellt durch Umsetzung eines geeigneten reaktionsfähigen Derivats einer N-Acylaminomalonsäure mit einem entsprechenden Amin oder aber es wird zuerst ein Monoamid der entsprechenden N-Acylaminomalonsäure hergestellt und diese als solche, oder indem man es nochmals in eine aktive Form überführt, mit einem weiteren Amin zu einem Diamid der allgemeinen Formel (V) umgesetzt.

Für die Herstellung der N-Acylmalonsäurederivate der allgemeinen Formel V lassen sich die gleichen Methoden anwenden, wie die oben für die Darstellung der Carboxamide der allgemeinen Formel I beschrieben wurden.

Bevorzugt wird auch hier die Methode der aktiven Imidazolide.

BEISPIEL 1

5,6-Dihydro-8,9-dimethoxy-3-methyl-imidazo-[1,5-a]-isochinolin-1-carbonsäureethylester

50 g 2-(3,4-Dimethoxyphenyl)-ethylaminocarobnyl-N-acetyl-glycinethylester werden unter Rühren in 100 g eines auf 100°C vorgewärmten Methansulfonsäure/$P_2O_5$-Gemisches - (100:10) eingetragen und ca. 30 Minuten bei ca. 120°C belassen. Nach beendeter Umsetzung wird die Reaktionsmischung aufeinanderfolgend auf Eis gegossen, mit Sodalösung neutralisiert, mit $CH_2Cl_2$ extrahiert, über $Na_2SO_4$, getrocknet, auf ca. 50 ml eingeengt mit ethanolischer Salzsäure versetzt, und unter Zugabe von Diethylether wird das Hydrochlorid (Fp. 214°C) gefällt.

Ausbeute: 38,2 g (85 % der Theorie)

BEISPIEL 2

5,6-Dihydro-8,9-dimethoxy-3-methyl-imidazo-[1,5-a]-isochinolin-1-carbonsäure

25,9 g 5,6-Dihydro-8,9-dimethoxy-3-methyl-imidazo-[1,5-a]-isochinolin-1-carbonsäureethylester werden in einer Lösung von 5,0 g KOH in Ethanol bei ca. 60°C verseift. Nach beendeter Reaktion wird die Reaktionsmischung aufeinanderfolgend mit ethanolischen HCl neutralisiert, auf 0°C abgekühlt, ausgefallenes KCl abgesaugt, das Filtrat eingeengt, das Hydrochlorid (Fp. 214°C) gebildet und aus Ethanol kristallisiert.

Ausbeute: 22,6 g (96 % der Theorie)

BEISPIEL 3

5,6-Dihydro-8,9-dimethoxy-3-methyl-imidazo-[1,5-a]-isochinolin-1-(N-methyl)-carboxyamid

Variante A

Ein Gemisch aus 10 g 2-(3,4-Dimethoxyphenyl)-ethylamino-carbonyl-N-acetyl-glycin-N-methylamid, 100 g Acetonitril und 30 ml Phosphoroxychlorid wird 1-2 Stunden unter Rückfluß erhitzt. Nach beendeter Umsetzung wird die Reaktionsmischung im Vakuum eingeengt, mit gesättigter Sodalösung alkalisiert, das Reaktionsprodukt in üblicher Weise isoliert, an Kieselgel - ($CH_2Cl_2$:MeOH 100:5) gereinigt und als Hydrochlorid (Fp. 208°C Ethanol) kristallisiert.

Ausbeute: 4,2 g (40 % der Theorie)

Variante B

Ein Gemisch aus 16 g 2-(3,4-Dimethoxyphenyl)-ethylaminocarbonyl-N-acetylglycin-N-methylamid und 60 g Polyphosphorsäure wird ca. 1/2 Stunde auf 130°C erhitzt. Nach beendeter Reaktion läßt man Reaktionsmischung auf Raumtemperatur abkühlen, gießt auf Eis und stellt mit konzentrierter $NH_3$-Lösung alkalisch. Das Umsetzungsprodukt wird durch Ausschütteln $CH_2Cl_2$ isoliert, über Kieselgel gereinigt (Eluent: $CH_2Cl_2$/MeOH, 100:5) und das Hydrochlorid (Fp. 208-209°C) überführt.

Ausbeute: 7,2 ( 50 % der Theorie)

Variante C

5 g 5,6-Dihydro-8,9-dimethoxy-3-methyl-imidazo-[1,5-a]-isochinolin-1-carbonsäurehydrochlorid werden in 40 ml wasserfreiem Dimethylformamid bei Raumtemperatur mit 3,1 g N,N'-Carbonyldiimidazol 30 Minuten gerührt und darauf mit 10 ml einer gesättigten Lösung von Methylamin in $CHCl_3$ versetzt. Nach 1 Stunde Rühren, wird das Lösungsmittel im Vakuum abgezogen, das Produkt zwischen $CH_2Cl_2$ und Wasser verteilt, die organische Phase über $Na_2SO_4$ getrocknet und im Vakuum eingedampft. Zur Bildung des Hydrochlorids wird der Rückstand in wenig wasserfreiem Ethanol gelöst, mit ethanolischer Salzsäure versetzt und danach das Hydrochlorid (Fp. 209°C) durch Zugabe von Ether zur Kristallisation gebracht.

Ausbeute 5,4 g (93 % der Theorie)

Variante D

10 g 5,6-Dihydro-8,9-dimethoxy-3-methyl-imidazo-[1,5-a]-isochinolincarbonsäuremethylester werden in 200 ml einer mit Methylamin gesättigten Chloroformlösung 24 Stunden bei Raumtemperatur

gerührt. Nach beendeter Umsetzung wird die Reaktionsmischung im Vakuum eingedampft und das Umsetzungsprodukt in das Hydrochlorid (Fp. 208°C) überführt.

Ausbeute: 8,0 g (81 % der Theorie).

Analog der in den Beispielen 1 bis 3 angegebenen Arbeitsweisen wurden ferner die folgenden Endprodukte sythetisiert:

TABELLE 1

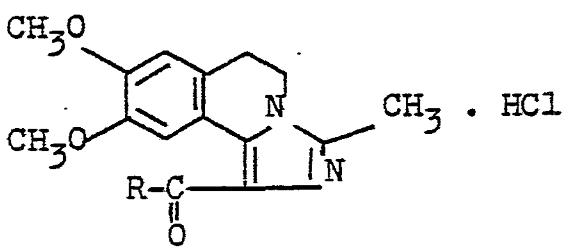

| Beispiel | R | Fp°C | Ausbeute %der Theorie |
|---|---|---|---|
| 4 | $-OCH_3$ | 197-202 | 77 |
| 5 | $-NH_2$ | 215 | 85 |
| 6 | $-NHC_2H_5$ | 180-183 | 79 |
| 7 | $-NH(CH_2)_2-CH_3$ | 219-222 | 68 |
| 8 | $-NH(CH_2)_3-CH_3$ | 212-214 | 70 |
| 9 | $-NH(CH_2)_4CH_3$ | 204 | 71 |
| 10 | $-NH-CH(CH_3)_2$ | >250 | 66 |
| 11 | $-NH-CH_2-CH(CH_3)_2$ | 228-229 | 64 |
| 12 | $-NH(CH_2)_2-CH(CH_3)_2$ | 208-220 | 71 |

| | | | |
|---|---|---|---|
| 13 | $-NHCH_2CH=CH_2$ | 216 | 67 |
| 14 | $-NHCH_2C\equiv CH$ | 189 | 57 |
| 15 | $-NH-CH_2-$ 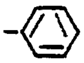 | 239-241 | 61 |
| 16 | $-NHCH_2-$ 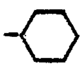 | 228-230 | 72 |
| 17 | -NH- 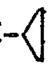 | 208-210 | 69 |
| 18 | -NH- 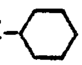 | 237-238 | 61 |
| 19 | $-NH-(CH_2)_2OCH_3$ | 219-224 | 74 |
| 20 | $-NH-(CH_2)_3OCH_3$ | 203 | 67 |
| 21 | $-NH(CH_2)_2-OH$ | 155 | 68 |
| 22 | $-NH(CH_2)_3-OH$ | 164-165 | 64 |
| 23 | $-NH(CH_2)_2Cl$ | 217 | 46 |
| 24 | $-NH(CH_2)_3Cl$ | 218 | 43 |
| 25 | $-NH(CH_2)_2-N(CH_3)_2$ | 215 | 68 |
| 26 | $-NH(CH_2)_2-NH-$ 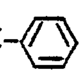 | 214 | 63 |
| 27 | $-NH(CH_2)_2-$ 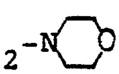 | 239-240 | 70 |
| 28 | $-NH-CH_2-$ 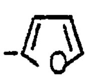 | 226 | 73 |
| 29 | $-NH-(CH_2)_2-$ 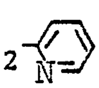 | 270-273 | 68 |

| | | | |
|---|---|---|---|
| 30 | $-NH-(CH_2)_2-$ 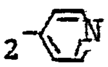 | 244-246 | 63 |
| 31 | $-NH-(CH_2)_2-$ 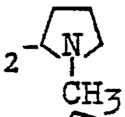 | 155(Zerstört) | 64 |
| 32* | $-NH-(CH_2)_2-$ 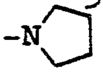 | 96 | 67 |
| 33 | $-NH-CH_2-$ 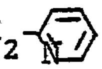 | 221 | 63 |
| 34 | -NH- 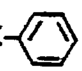 | 176-179 | 59 |
| 35 | -NH- 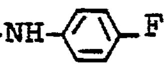 | 242-243 | 67 |
| 36 | -NH- 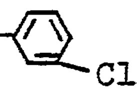 | 212(Zerstört) | 70 |
| 37 | $-N(CH_3)_2$ | 244-246 | 59 |
| 38 | 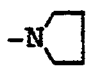 | 228-230 | 53 |
| 39 | 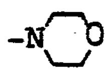 | 262-263 | 55 |
| 40 | $-NH(CH_2)_3-N(CH_3)_2$ | 239-241 | 67 |
| 41 | 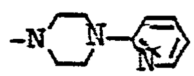 | 175 | 63 |
| 42 | 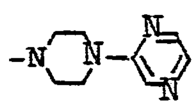 | 153-154 | 65 |
| 43 | 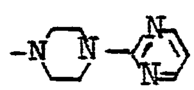 | 210-212 | 67 |
| 44 | -NH- 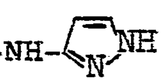 | 260 | 58 |
| 45 | -NH-NH- 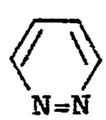 -Cl | 219-220 | 57 |

*Die Verbindung von Beispiel 32 wurde als Freibase 1 erhalten. Fp. und die Ausbeute gelten für die Basen.

TABELLE 2

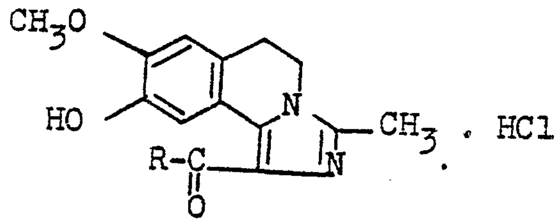

| Beispiel | R | Fp°C | Ausbeute % d. Th. |
|---|---|---|---|
| 46 | $-OC_2H_5$ | 238-239 | 62 |
| 47 | $-NH-(CH_2)_2-OCH_3$ | >250 | 59 |
| 48 | $-NH(CH_2)_2-$ 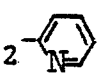 | >250 | 61 |

TABELLE 3

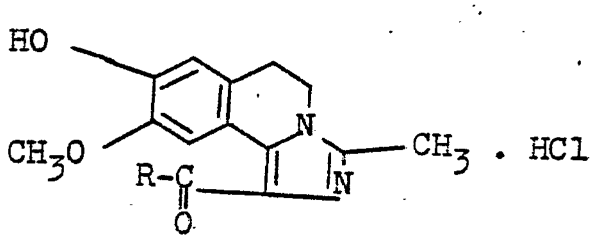

| Beispiel | R | Fp°C | Ausbeute % d. Th. |
|---|---|---|---|
| 49 | $-NH-(CH_2)_2OCH_3$ | 198-199 | 56 |

TABELLE 4

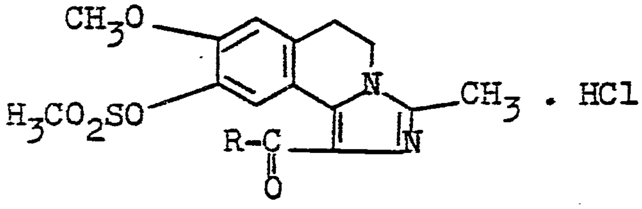

| Beispiel | R | Fp°C | Ausbeute % d. Th. |
|---|---|---|---|
| 50 | $-OC_2H_5$ | 210-211 | 67 |

TABELLE 5

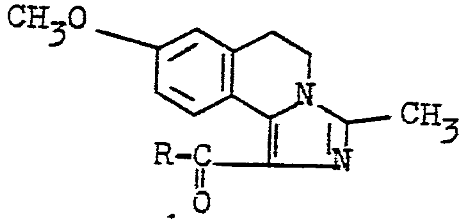

| Beispiel | R | Fp°C | Ausbeute % d. Th. |
|---|---|---|---|
| 51 | $-OC_2H_5$ | 201-203 | 87 |
| 52 | $-NH-(CH_2)_2-$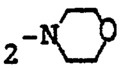 | 148-149 | 65 |
| 52(a) | $-NHCH_2CH(CH_3)_2$ | 184-186 | ---- |

TABELLE 6

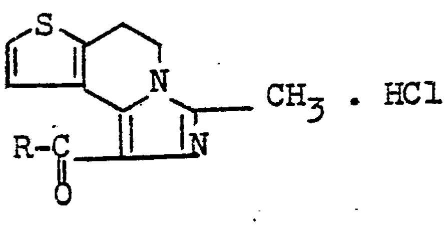

| Beispiel | R | Fp°C | Ausbeute % d. Th. |
|---|---|---|---|
| 53 | $-OC_2H_5$ | 198-199 | 78 |
| 54 | $-NH(CH_2)_2OCH_3$ | 156-158 | 63 |

TABELLE 7

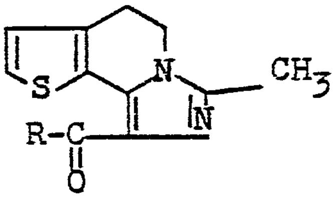

| Beispiel | R | Fp.°C | Ausbeute % d. Th. |
|---|---|---|---|
| 55 | $-OC_2H_5$ | 208-210 | 67 |
| 56 | $-NH(CH_2)_2OCH_3$ | 208 | 62 |
| 57 | $-NH(CH_2)_2-$ 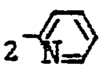 | >250 | 61 |
| 58 | $-NH(CH_2)_2-$ 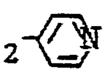 | 245-250 | 58 |
| 59 | $-NH-CH_2-CH=CH_2$ | 274 | 73 |
| 60 | $-NH-(CH_2)_2-$ 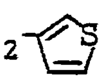 | 226-229 | 75 |

## Pharmazeutische Formulierungsbeispiele

a) Tabletten

| | pro Tablette |
|---|---|
| Wirkstoff | 25 mg |
| Milchzucker | 140 mg |
| Maisstärke | 240 mg |
| Polyvinylpyrrolidon | 15 mg |
| Magnesiumstearat | 5 mg |
| | 425 mg |

Der feingemahlene Wirkstoff, Milchzucker und ein Teil der Maisstärke werden miteinander vermischt. Die Mischung wird gesiebt, worauf man sie mit einer Lösung von Polyvinylpyrrolidon in Wasser befeuchtet, knetet, feuchtgranuliert und trocknet. Das Granulat, der Rest der Maisstärke und das Magnesiumstearat werden gesiebt und miteinander vermischt. Das Gemisch wird zu Tabletten geeigneter Form und Größe verpreßt.

b) Tabletten

| | pro Tablette |
|---|---|
| Wirkstoff | 15 mg |
| Maisstärke | 190 mg |
| Milchzucker | 55 mg |
| Mikrokristalline Cellulose | 35 mg |
| Polyvinylpyrrolidon | 15 mg |
| Natrium-carboxymethylstärke | 23 mg |
| Magnesiumstearat | 3 mg |
| | 335 mg |

Der feingemahlene Wirkstoff, ein Teil der Maisstärke, Milchzucker, mikrokristalline Cellulose und Polyvinylpyrrolidon werden miteinander vermischt, die Mischung gesiebt und mit dem Rest der Maisstärke und Wasser zu einem Granulat verarbeitet, welches getrocknet und gesiebt wird. Dazu gibt man die Natrium-carboxymethylstärke und das Magnesiumstearat, vermischt und verpreßt das Gemisch zu Tabletten geeigneter Größe.

c) Ampullen

| | | |
|---|---|---|
| Wirkstoff | | 10,0 mg |
| Natriumchlorid | | 10,0 mg |
| bidestilliertes Wasser | q.s. ad | 1,0 ml |

Herstellung

Der Wirkstoff und das Natriumchlorid werden in bidestilliertem Wasser gelöst und die Lösung in Ampullen steril abgefüllt.

d) Tropfen

| | | |
|---|---|---|
| Wirkstoff | | 1,0 mg |
| p-Hydroxybenzoesäuremethylester | | 0,1 mg |
| p-Hydroxybenzoesäurepropylester | | 0,1 mg |
| entmineralisiertes Wasser | q.s. ad | 100,0 ml |

Herstellung:

Der Wirkstoff und die Konservierungsmittel werden in demineralisiertem Wasser gelöst und die Lösung filtriert und in Flaschen zu je 100 ml abgefüllt.

## Ansprüche

1. Verbindungen der allgemeinen Formel I

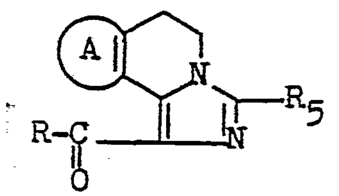

I

in der

A eine Gruppe bedeutet, die zusammen mit den zwei Kohlenstoffatomen des Pyridylsystems, an welche sie gebunden ist, eine ein-oder mehrfach substituierte Phenyl-oder eine ein-oder mehrfach substituierte Thienogruppe bildet, wobei der oder die Substituenten eine $C_1$-$C_3$Alkoxy, Hydroxy oder Methansulfonyloxygruppe darstellen;

$R_5$ bedeutet eine $C_1$-$C_4$Alkylgruppe; und

R bedeutet

(i) -OH,

(ii) eine gesättigte oder ungesättigte, verzweigte oder unverzweigte $C_1$-$C_5$Alkoxygruppe; oder

(iii) -$NR_1R_2$, worin $R_1$ und $R_2$ unabhängig voneinander stehen für

a) Wasserstoff

b) eine gesättigte oder ungesättigte, verzweigte oder unverzweigte Kohlenwasserstoffgruppe mit 1 bis 8 Kohlenstoffatom-(en) (gegebenenfalls ein oder mehrfach substituiert durch Halogen, Hydroxy, Methoxy, Amino, Methylamino, Anilino, Dimethylamino, $C_3$-$C_7$ Cycloalkyl, Phenyl - (gegebenenfalls ein-oder mehrfach substutiert durch Halogen oder Methoxy) oder einen heterocyclischen 5-bis 7-Ring, der ein oder zwei gleiche oder verschiedene Heteroatome aus der Gruppe N, S und O enthält, und gegebenenfalls durch ($C_1$-$C_3$)alkyl substituiert ist);

c) eine $C_3$-$C_7$ Cycloalkylgruppe;

d) Phenyl, gegebenenfalls ein oder mehrfach durch Halogen oder Methoxy substituiert:

e) einen heterocyclischen 5-bis 7-Ring, der ein oder zwei gleiche oder verschiedene Heteroatome, aus der Gruppe N, S oder O, enthält;

f) Amino substituiert durch einen heterocyclischen 5-bis 7-Ring, der ein oder zwei gleiche oder verschiedene Heteroatome, ausgewählt aus der Gruppe N, S oder O, enthält, und der gegebenenfalls ein oder mehrfach durch Halogen substituiert ist,

oder $R_1$ und $R_2$ bilden zusammen mit dem Stickstoffatom einen 5-oder 6-Ring, der gegebenenfalls ein Sauerstoffatom oder ein weiteres Stickstoffatom als Ringheteroatom enthält, und der seinerseits durch einen 5-oder 6-gliedrigen homo- oder heterocyclischen Ring, der ein oder zwei gleiche oder verschiedene Heteroatome, aus der Gruppe N, S und O enthält, substituiert sein kann.

2. Verbindungen gemäß Anspruch 1, der allgemeinen Formel II

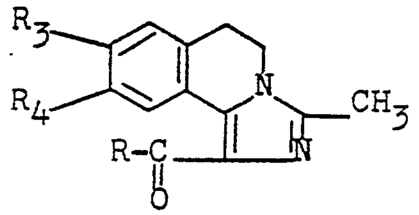

II

worin $R_3$ und $R_4$ unabhängig voneinander Wasserstoff, Hydroxy, Methoxy und Methansulfonyloxy bedeuten, und R wie in Anspruch 1 angegeben ist, und ihre Säureadditionssalze.

3. Verbindungen gemäß Anspruch 2, worin $R_3$ und $R_4$ Methoxy sind.

4. Verbindungen gemäß Anspruch 2, worin $R_3$ Methoxy ist und $R_4$ Hydroxy ist oder umgekehrt.

5. Verbindungen gemäß Anspruch 2, worin $R_3$ Methoxy und $R_4$ Methansulfonyloxy ist oder umgekehrt.

6. Verbindungen gemäß Anspruch 2, worin $R_3$ Methoxy und $R_4$ Wasserstoff ist oder umgekehrt.

7. Verbindungen gemäß Anspruch 1 der allgemeinen Formel III

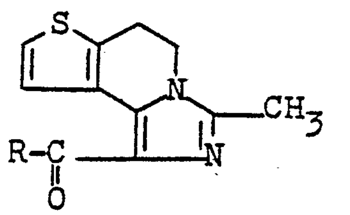

III

oder Formel IV

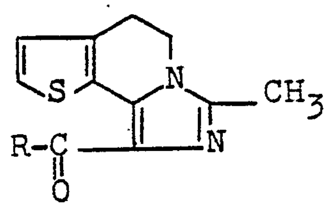

IV

worin R wie in Anspruch 1 angegeben ist, und ihre Säureadditionssalze.

8. Verbindungen gemäß einem der Ansprüche 1 bis 7, worin R für Methoxy oder Ethoxy steht.

9. Verbindungen gemäß einem der Anspruche 1 bis 7, worin R die Gruppe $-NR_1R_2$ bedeutet, wobei $R_1$ für Wasserstoff und $R_2$ für eine $C_1$-$C_5$-Alkyl-, eine $C_3$-$C_5$-Alkenyl-, eine $C_3$-$C_5$-Alkinyl-, eine $C_3$-$C_6$-Cycloalkyl($C_1$-$C_5$)-alkyl oder eine $C_3$-$C_7$-Cycloalkylgruppe stehen, oder $R_1$eine und $R_2$ je für eine $C_1$ - $C_2$-Alkylgruppe stehen.

10. Verbindungen gemäß einem der Ansprüche 1 bis 7, worin R die Gruppe $-NR_1R_2$ bedeutet, wobei $R_1$ für Wasserstoff und $R_2$ für eine $C_1$-$C_5$-Alkylgruppe steht, die substituiert ist durch

a) einen heterocyclischen 5-oder 6-Ring enthaltend zumindest eine Doppelbindung und mindestens ein N, S oder O Heteroatom; oder

b) einen gegebenenfalls N-Methylsubstituierten heterocyclischen 5-oder 6-Ring ohne Doppelbindung, welcher ein O Atom als weiteres heteroatom enthalten kann, und ihre Säureadditionssalze.

11. Verbindungen gemäß einem der Ansprüche 1 bis 7, worin R die Gruppe $-NR_1R_2$ bedeutet, wobei $R_1$ für Wasserstoff und $R_2$ für eine $C_1$-$C_5$-Alkylgruppe steht, die substituiert ist durch Phenyl, Hydroxy, Methoxy, Chlor, Dimethylamino oder Anilino und ihre Säureadditionssalze.

12. Verbindungen gemäß einem der Ansprüche 1 oder 7, worin R die Gruppe $-NR_1R_2$ bedeutet, wobei

$R_1$ und $R_2$ zusammen mit dem Stickstoffatom eine Piperazingruppe bilden, welche durch einen heterocyclischen 6-Ring, der ein oder zwei N-Atome enthält, N-substituiert ist.

13. Verbindungen gemäß einem der Ansprüche 1 bis 7, worin R die Gruppe $-NR_1R_2$ bedeutet, wobei $R_1$ und $R_2$ zusammen mit dem Stickstoffatom einen heterocyclischen Ring ohne Doppelbindung, welcher gegebenenfalls als weiteres Heteroatom ein N oder O-Atom enthält, bilden.

14. Die Verbindung 5,6-Dihydro-8,9-dimethoxy-3-methyl-imidazo-[1,5-a]-isochinolin-1-(N-methyl)-carboxyamid;

5,6-Dihydro-8,9-dimethoxy-3-methyl-imidazo-[1,5-a]-isochinolin-1-(N-ethyl)-carboxyamid;

5,6-Dihydro-8,9-dimethoxy-3-methyl-imidazo-[1,5-a]-isochinolin-1-[N-n-pentyl]-carboxyamid;

5,6-Dihydro-8,9-dimethoxy-3-methyl-imidazo-[1,5-a]-isochinolin-1-[N-(2-methoxy-ethyl)]-carboxyamid;

5,6-Dihydro-8,9-dimethoxy-3-methyl-imidazo-[1,5-a]-isochinolin-1-[N-2-(4-morpholinyl)ethyl]-carboxyamid;

5,6-Dihydro-8-methoxy-3-methyl-imidazo-[1,5-a]-isochinolin-1-[N-isobutyl]-carboxyamid;

5,6-Dihydro-8-methoxy-3-methyl-imidazo-[1,5-a]-isochinolin-1-carbonsäureethylester;

5,6-Dihydro-imidazo[1,5-a]-thieno[3,2-c]-pyridin-1-[N-2-(methoxy-ethyl)]-carboxyamid, oder ihre Säureadditionssalze.

15. Verfahren zur Herstellung von Verbindungen gemäß Anspruch 1 bis 14, dadurch gekennzeichnet, daß man

A) (zur Herstellung von Verbindung der allgemeinen Formel I in welcher R nicht-$NH_2$ ist) eine Verbindung der allgemeinen Formel V

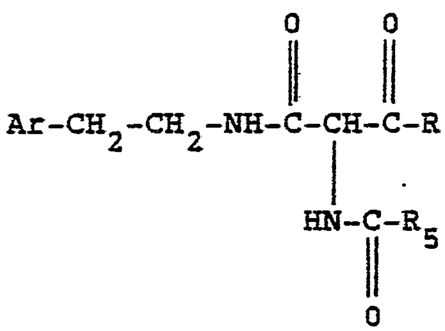

V

worin Ar für eine unsubstituierte oder ein oder mehrfach substituierte Phenylgruppe oder eine unsubstituierte oder ein-oder zweifach substituierte Thienogruppe steht, mit der Maßgabe, daß eine -CH=Gruppe der Phenyl-oder Thienogruppe, die dem Kohlenstoffatom benachbart ist, das die Seitenkette trägt, unsubstituiert ist, und wobei das oder die Substituenten aus der Gruppe $C_1$-$C_3$-Alkoxy, Hydroxy oder Methansulfonyloxy ausgewählt sind und R die oben angegebenen Bedeutungen hat, in Gegenwart eines geeigneten Kondensationsmittels cyclisiert.

B (zur Herstellung von Verbindungenen gemäß Anspruch 1, worin R $-NR_1R_2$ ist) eine Verbindungen der allgemeinen Formel VI

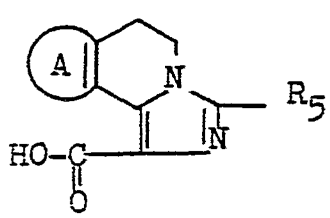

VI

oder ein ($C_1$-$C_5$)Alkylester oder ein aktiviertes Derivat davon mit einer Verbindungen der allgemeinen Formel V

HNR $_1$R $_2$

umsetzt,

und gewünschtenfalls eine so erhaltene freie Base (von Verfahren A oder B) in ein physiologisch unbedenkliches Säureadditionssalz überführt, beziehungsweise aus einem bei der Cyclisierung erhaltenen Salz die Base freisetzt.

16. Verwendung einer Verbindung gemäß einem der Ansprüche 1 bis 14, zur Behandlung von Coronarerkrankungen.

17. Verwendung einer Verbindungen gemäß einem der Ansprüche 1 bis 14 zur Behandlung von Angina pectoris.

18. Verwendung einer Verbindungen gemäß einem der Ansprüche 1 bis 14 zur Verbesserung der Ischämie-Toleranz im Myocard.

19. Verwendung einer Verbindungen gemäß einem der Ansprüche 1 bis 14 zur Verbesserung der Gewebsdurchblutung oder die Gewebesauerstoffversorgung, insbesondere im zentralen Nervensystem, und zur Behandlung der Zustandsformen eingeschränkter cerebraler Leitstungsfähigkeit.

20. Arzneimittel, enthaltend als Wirkstoff eine der mehrere Verbindungenen gemäß einem der Ansprüche 1 bis 14.

21. Eine Verbindungen der Formel

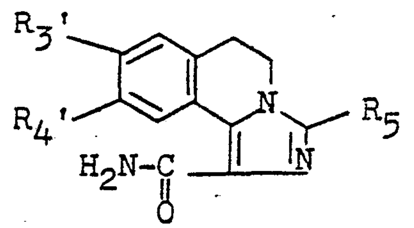

VIII

und ihre Säureadditionssalze, worin $R_3$' und $R_4$' unabhängig voneinander Hydroxy oder Methoxy bedeuten, zur Behandlung von Coronarerkrankungen oder zur Verbesserung der Gewebedurchblutung und Gewebesauerstoffversorgung, insbesondere im zentralen Nervensystem und zur Behandlung der Zustandsformem eingeschränkter cerebraler Leistungsfähigkeit.

22. Eine Verbindungen gemäß Anspruch 21, zur Behandlung von Angina pectoris oder zu Verbesserung der Ischämie-Toleranz im Myocard.

23. Verwendung einer Verbindungen gemäß Formel VIII zur Herstellung von Arzneimitteln zur Behandlung von Coronarerkrankungen oder zur Verbesserung der Gewebedurchblutung, insbesondere im zentralen Nervensystem und zur Behandlung der Zustandsformen eingeschränkter cerebraler Leistungsfähigkeit.

24. Verwendung einer Verbindungen gemäß Formel VIII zur Herstellung von Arzneimitteln zur Behandlung von Angina pectoris oder zur Verbesserung der Ischämie-Toleranz im Myocard.